# EUROPEAN PATENT APPLICATION

(11) **EP 3 632 409 A1**
(43) Date of publication of application: **08.04.2020**
(21) Application number: 18805082.7
(22) Date of filing: 26.04.2018
(51) Int. Cl.: A61K 8/99, A61Q 19/02

(54) **USE OF EXOSOME IN SKIN WHITENING PREPARATION**

(30) Priority: 23.05.2017 CN 201710370493
(71) Applicant: Beijing Sinocell Co., Ltd, Beijing 100176 (CN)
(72) Inventor: LI, Zongjin, Beijing 100176 (CN); HAN, Zhihai, Beijing 100176 (CN)
(74) Representative: Schmid, Wolfgang
(86) International application number: PCT/CN2018/084668
(87) International publication number: WO 2018/214694

(57) **Abstract**

The present invention provides the application of mesenchymal stem cell-derived exosomes in a skin whitening preparation, the mesenchymal stem cell is originated from human umbilical cord or placenta.

## Description

### TECHNICAL FIELD

The present invention relates to the field of stem cells, in particular to the application of highly active exosome produced by stem cells in skin whitening preparation.

### BACKGROUND ART

Exosome is a vesicle-like body secreted by cells extracellularly, which has a diameter between 30-150 nm and has a typical lipid bilayer membrane structure. It may be present in cell culture supernatant, plasma, serum, saliva, urine, amniotic fluid, and malignant ascites, as well as other biological fluids; it carries important information such as various proteins, lipids, DNA, and RNA of the parent cell, and plays an important role in inter-cellular transmission of material and information.

Exosomes of various cell origins are involved in important physiological and pathological processes such as antigen presentation, genetic material exchange, immune response, angiogenesis, inflammation, tumor metastasis and treatment, pathogen or oncogene transmission. These functions are dependent on the contents of the exosome, especially proteins and RNA.

Current studies have demonstrated that exosomes secreted by mesenchymal stem cells play an important role in promoting skin damage repair, reducing inflammation, and promoting angiogenesis.

Related studies suggest that exosomes from human umbilical cord mesenchymal stem cells mediate the WNT4 signaling pathway and participate in the repair of skin wounds. In mice, human umbilical cord mesenchymal stem cell-mediated Wnt/b-catenin activity plays an important role in epithelialization and cell proliferation. When exosome Wnt4 from the human umbilical cord mesenchymal stem cells is knocked out, b-catenin activity disappears, and the skin repair effect *in vivo* decreased significantly. This indicates that stem cell plays an effect on skin repair through exosome.

HAN Weidong from the General Hospital of the People's Liberation Army used endotoxin to pre-treat mesenchymal stem cell to enhance its paracrine effect, and improve the nutritional support and regeneration and repair performance of stem cell. The study found that mesenchymal stem cells, by releasing a large number of exosomes, construct a static microenvironment of tissue repair, therefore eliminate inflammation and promote wound healing.

Researchers from the Institute of Hematology have studied that exosome from human bone marrow mesenchymal stem cells has immune-modulatory functions and roles of support for angiogenesis. The exosome secreted by normal human bone marrow mesenchymal stem cells inhibits human peripheral blood mononuclear cells to secrete IFN-γ, comprising the immune-related microRNA such as miR301, miR22, miR-let-7a, and the like, and it can promote the network formation of human umbilical vein endothelial cells and blood vessel formation. Angiogenesis is a process in which endothelial cells proliferate and migrate to form small blood vessels. The exosome miR-92 derived from K562 cells can promote the migration and tubular structure formation of human umbilical vein endothelial cells, therefore promote angiogenesis. If the exosome can act on the human skin to promote angiogenesis, it will transport nutrients to the skin, transport metabolic waste, and make the skin younger.

Korean scientists have studied that adipose-derived stem cells and their secretory factors have a wrinkle-removing effect on UV-induced wrinkles, and the wrinkle-removing effect is mainly achieved by reducing the apoptosis induced by UVB and by promoting the synthesis of collagen by human fibroblasts.

In recent years, with the pursuit of skin whitening, more and more people use whitening skin care products. The melanin in the skin is synthesized by melanocytes, which diffuse to adjacent cells by osmosis and form color on the skin. The color depth is mainly determined by the amount of melanin in the skin. The biochemical reaction process in melanocytes is that: fatty acid tyrosine produces dopaquinone under the action of tyrosinase, which is converted to melanin by red pigment and leuco pigment under the action of enzyme or non-enzymatic oxidation. This process is the main cause of melanin. Therefore, by hindering or inhibiting the action of tyrosinase in the first stage of the reaction, it is possible to inhibit melanogenesis, so discovering a drug capable of hindering or inhibiting the action of tyrosinase is extremely important to prevent or inhibit the production of melanin by tyrosinase.

It is an object of the present disclosure to provide use of highly active exosome produced by stem cells in skin whitening formulations.

### SUMMARY OF THE DISCLOSURE

A first technical solution of the present disclosure is the use of a mesenchymal stem cell-derived exosome in a skin whitening preparation.

The second technical solution is based on the first technical solution, characterized in that, the mesenchymal stem cells are human mesenchymal stem cells.

The third technical solution is based on the second technical solution, characterized in that, the mesenchymal stem cells are originated from human perinatal umbilical cord or placenta.

The fourth technical solution is based on the third technical solution, characterized in that, the concentration of exosomes is 800 µg/ml or more.

The fifth technical solution is based on the fourth technical solution, characterized in that, the skin whitening preparation is an externally applied preparation or preparation for local injection or microneedle injection.

The sixth technical solution is based on the fifth technical solution, characterized in that, the mesenchymal stem cells are prepared by the following method:
Take the placenta of the full-term fetus delivered by caesarean section, the chorion from the placenta is separated under sterile conditions, RBCs are rinsed off with PBS, the chorion is cut into tissue blocks with a size of 10-20 mm³, to which collagenase digestion solution is added, and is magnetically stirred and digested at a temperature around 37°C for a period of time; after the digested ,tissue suspension is filtered through a 100 mesh filter, the filtrate is added to a centrifuge tube containing a small amount of 1×PBS, and the remaining tissue is further magnetically stirred and digested by trypsin at a temperature around 37°C for a certain period of time, the digested suspension is filtered through a 100-mesh filter, and the filtrate is collected into a centrifuge tube, to which serum is added to terminate the digestion; after the tissue block has almost been digested completely, it is centrifuged, the supernatant is discarded carefully, and the cells obtained by centrifugation are collected; some cell samples are taken and counted, then inoculated to DMEM-LG medium containing 10% FBS at a density of 2×10⁶/cm²; after cultivation in an incubator at a temperature around 37°C, saturated humidity, and 5% CO₂ for several days, culturing medium is replaced for the first time, to remove unattached cells; then medium is changed in a regular interval, and the morphology and growth of the cells are observed under the microscope daily; when a confluence of 80% to 90% is reached, the digested cells are subcultured;
the mesenchymal stem cell derived-exosomes are prepared by the following methods:
   3-6 passages of human placenta mesenchymal stem cells are cultured in DMEM medium supplemented with 10% fetal bovine serum; when the cell confluence reaches 70-80%, cell cultivation continues till the cell confluence reaches 90%; supernatant is collected then centrifuged at 2000G for 20 min to remove cells and debris, then filtered through a 0.22 µm sterile filter into a centrifuge tube, then polyethylene glycol 8000 solution with a final concentration of 10% is added into the centrifuge tube, vortexed and mixed homogenously, and incubated at 4°C overnight; then after centrifugation at 4000G for 20 min, supernatant is poured out, and sediment is resuspended in PBS solution with a volume of 1/100-1/50 of that of original supernatant, then is incubated in the polyethylene glycol of a final concentration of 8% for 1h again, and after centrifugation, sediment is resuspended in PBS again, dispensed and stored at -80°C.

As a skin whitening preparation, the obtained exosomes can be used for local injection, or hyaluronic acid and the like can be added for microneedle injection, or for external application onto the skin.

### BRIEF DESCRIPTION OF THE DRAWINGS

Figure 1 is an identification flow chart of human placental chorionic mesenchymal stem cells;
Figure 2 is a diagram showing the protein distribution of exosomes after 10% polyacrylamide gel electrophoresis;
Figure 3 is a CD81 phenotype identification map of exosomes enriched by CD63+ magnetic beads;
Figure 4 is a graph showing the concentration of cytokines HGF and bFGF in exosomes;
Figure 5 shows the effect of exosomes on intracellular melanin content.

### DETAILED DESCRIPTION

The embodiments of the present disclosure will be described in details below with reference to the accompanying drawings.

Preparation of exosomes derived from placenta mesenchymal stem cells:

### (1) Isolation, cultivation and amplification of human perinatal mesenchymal stem cells:

The placenta of the full-term caesarean section fetus was taken and the chorion was mechanically separated from the placenta under sterile conditions. Care should be taken to prevent contamination of the cells from the basal decidua of mother. The RBCs were removed by rinsing with PBS (phosphate buffered saline). The chorionic tissue was cut into tissue blocks with a size of 10-20 mm³, collagenase digestion solution was added, and magnetic digestion was carried out at 37°C for 30 min. The digested tissue suspension was filtered through a 100 mesh screen and added to a centrifuge tube containing a small amount of 1 × PBS.

The filtered remaining tissue was further digested with trypsin at 37°C for 30 min, and the digested suspension was filtered through a 100 mesh filter. The filtrate was collected into a 50 ml centrifuge tube and serum was added to terminate digestion, at the same time the tissue block was almost digested completely. Then centrifugation at 3000 rpm for 15 min, the supernatant was discarded carefully, and the cells obtained by centrifugation were collected. A few cell samples were taken for counting, and inoculated to DMEM-LG medium containing 10% FBS at a density of 2×10⁶/cm², and cultivated in an incubator at a temperature 37°C, saturated humidity, and 5% CO₂. After 3 days, the medium was replaced for the first time, to remove unattached cells. The medium was changed every 3 to 4 days, and the morphology and growth of the cells were observed under the microscope daily. When the cell confluence reached 80% to 90%, the digested cells were subcultured.

### (2) Preparation of exosome-free serum medium

The fetal calf serum was centrifuged at 12,000×G for 12 hours at 4°C, and the sediment was discarded, and L-DMEM medium was added to obtain an exosome-free serum medium.

### (3) Preparation of mesenchymal stem cell supernatant which required by exosome

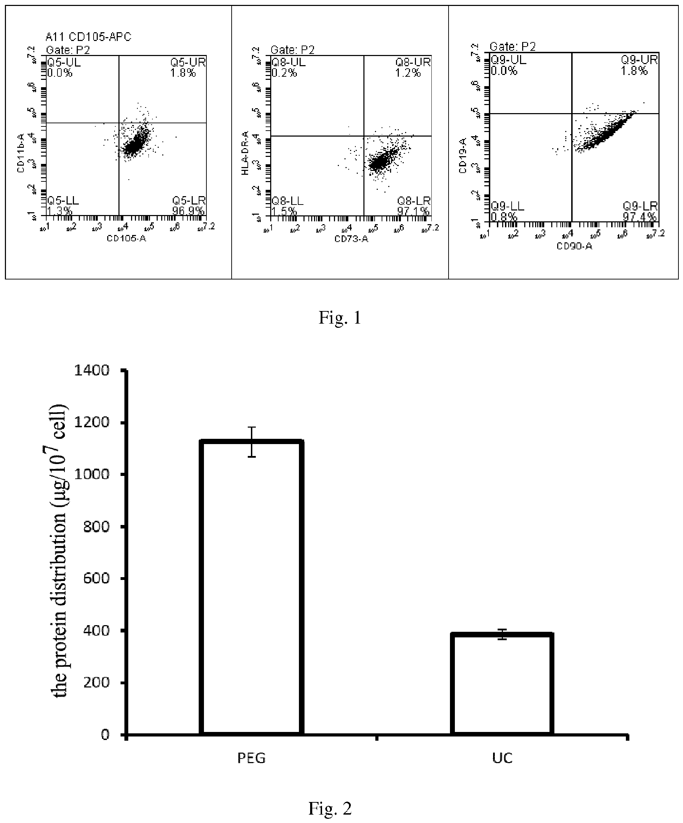

The strongly proliferative 2-5 passage of human placental mesenchymal stem cells in the logarithmic growth phase and in good state were selected and inoculated into the exosome-free serum medium (DMEM medium) at a rather high initial cell concentration. The cell confluence to be cultured reaches 90%. Figure 1 shows the identification flow chart of human placental chorionic mesenchymal stem cells. The results of cell phenotype identification are shown in Figure 1. Mesenchymal stem cells are positive for CD73, CD90, and CD105, and negative for CD11b, CD19, and HLA-DR.

Supernatant was collected and centrifuged at 2000G for 20 min to remove cells and debris, and then filtered through a 0.22 µm sterile filter into a centrifuge tube, and polyethylene glycol 8000 solution with a final concentration of 10% was added into the centrifuge tube. After vortexing and mixing homogenously, cultivation at 4°C for 12h, centrifugation at 4000G for 20 min, supernatant was poured out carefully, and sediment was resuspended in DPBS with a volume of 1/100 of volume of medium. Then resuspension was drawn into a 1.5 ml EP tube, to which polyethylene glycol 8000 solution with a final concentration of 8% was added, and incubated at 4°C for 4 h, followed by centrifugation at 10000 G for 10 min. Surface of the sediment was washed with DPBS, then the sediment was resuspended in 200 µl, dispensed at -80°C and stored.

For comparison, exosomes were separated by conventional ultracentrifugation as follows
Conventional ultracentrifugation method: 4°C, 2000×G, 10 min to remove debris of dead cells, supernatant was filtered at 0.22µm sterile filter to further remove impurities. After 120min of ultracentrifugation (at 4°C, 100000×G), supernatant was discarded, 50µl of PBS was added to wash again by 120 min of ultracentrifugation (at 4°C, 100000×g). Sediment was resuspended in 50 µl PBS, dispensed and stored at -80 °C.

BCA protein kit detected the protein content of the exosomes extracted by the above two methods. Polyethylene glycol (PEG) precipitation group is marked as PEG and ultracentrifugation precipitation group is marked as UC. Figure 2 shows the protein distribution of exosomes after 10% polyacrylamide gel electrophoresis. As shown in the figure, protein content of the exosomes obtained by PEG precipitation is significantly higher than that of the ultracentrifugation group.

The exosomes were analyzed as follows and the exosomes used for the analysis was those obtained by PEG precipitation.

Detection of successfully isolated exosomes from placental mesenchymal stem cells by using flow cytometric indicators
A magnetic bead coated with a CD63 antibody was used, which can bind to the CD63 antigen on the membrane surface of the exosome. The CD63+ magnetic beads were washed with 200 µl of 0.1% BSA solution and placed on the magnetic pole for 1 min, and the supernatant was discarded. The magnetic poles were removed and 100 µl of isolated and purified exosomes were added and stirred at 4°C in the refrigerator overnight. 300 µl of 0.1% BSA was added to wash with a pipette and mix homogenously. Placed on the magnetic pole for 1 min and supernatant was discarded. Then add 300 µl of isolation buffer , Magnetic beads was suspended in 300 µl of isolation buffer. Magnetic beads resuspended by 100 µl PBS which with 0.1% BSA in dispensing EP tube, 2 µl of CD81-PE were added, respectively, and the same type of IgG1-PE was used as a negative control. Incubation was carried out at RT for 45-60 min in the dark, and shaken gently from time to time. The positive expression rate of CD81 was detected by flow cytometry.

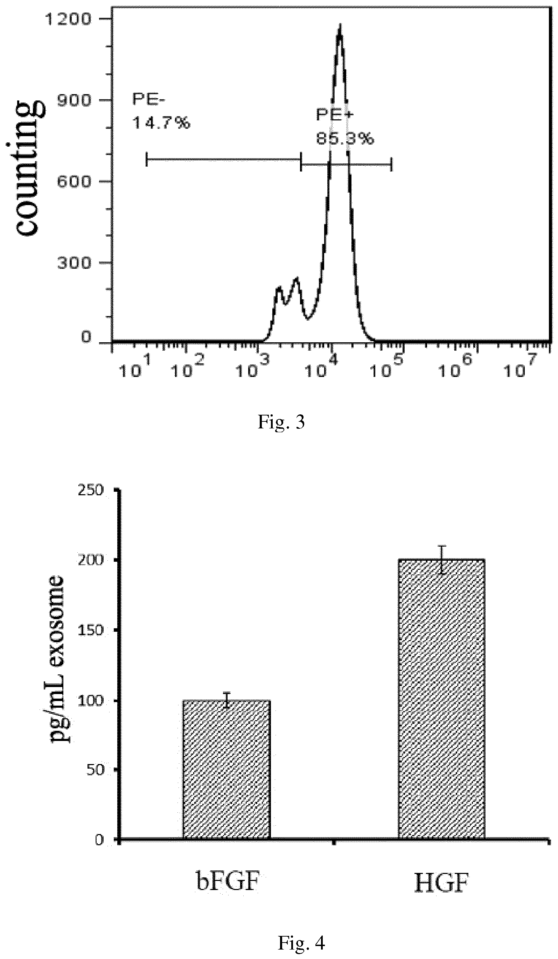

Figure 3 shows the CD81 phenotype identification of exosomes enriched by CD63+ magnetic beads. As shown in the figure, the positive expression rate of CD81 was 85.3%.

Elisa kit detected cytokine levels contained by exosomes.

Figure 4 is a graph showing concentration of cytokines HGF and bFGF contained in exosomes. As shown, the exosomes isolated by the method of the present embodiment contain higher level of hepatocyte growth factor (HGF) and basic fibroblast growth factor (bFGF).

The perinatal mesenchymal stem cells isolated successfully were detected by flow cytometric indicators, and the results showed that the isolation method in the present embodiment can obtain relatively pure mesenchymal stem cells, referring to the standard of mesenchymal stem cell phenotype proposed by the International Cell Therapy Association, the positive rate of CD73, CD90 and CD105 was not less than 95%; and the positive rate of CD19, CD34, CD45, CDllb and HLA-DR was not higher than 2%; the use of biological materials to stimulate human placenta mesenchymal stem cells can effectively improve the production of exosomes by at least one fold. The exosomes prepared by the chemical precipitation of the present embodiment had a protein concentration of 8,000-12,000 µg/ml as measured by BCA protein quantitative method, which was much higher than the ultracentrifugation method reported in the literature.

After magnetic beads enrichment and flow detection, 77.3% of the prepared exosomes could be captured by CD63+ magnetic beads, which proved a high purity. The positive expression rate of exosome CD81 in the flow detection control group (Exo) was 85.3%. The positive expression rate of exosome (NO-Exo) CD81 in the CS-NO environment was 89.8%.

It was found by gel protein electrophoresis that the exosomes obtained by the chemical precipitation of the present embodiment did not have excessive impurity proteins.

Evaluation of whitening effect of exosomes secreted by placental mesenchymal stem cells
(1) The effect of exosomes on the proliferation of melanocytes
   1) Experiment was carried on 4-5 passages of melanocytes. The cells in the logarithmic growth phase were selected and digested with 0.25% trypsin, the cell density was adjusted to 1×10⁴/ml with melanocyte culture medium, and every 100 µl was added to a 96-well culture plate, and each well was repeated for 5 wells.
   2) After 12 hours, the exosomes was diluted by culture medium, in which the concentration of exosomes was 100, 200, 400, 800, 1600 µg/ml, respectively, the original melanocyte culture medium in the culture plate was aspirated and discarded, then 100 µl of culture medium containing different concentrations of exosomes was added to each well.
   3) Incubated in a 5% CO₂ thermostatic incubator at 37°C for 24 h.
   4) 10 µl of CCK-8 solution was added.
   5) Incubation was continued for 2 hours in a cell culture incubator, and the absorbance was measured at 450 nm by an enzyme-linked immunosorbent assay. Cell viability = (Experimental group A - Blank group A) / (Control group A - Blank group A) × 100%.
(2) Effect of exosomes on melanin content in melanocytes
   1) Melanocytes in logarithmic phase were taken, culture medium was discarded, cells were washed with PBS solution twice, and cultured in 24-well plates.
   2) After 12 hours of culture, the cells were attached to the wall.
   3) The original medium was removed, and culture medium containing different concentrations of exosome was added. The control group and the blank control group were set, and three duplicate wells were set for each concentration group. Incubation was carried out in a 5% CO₂ thermostatic incubator at 37°C.
   4) Continued to culture for 72 h.
   5) The culture medium was aspirated, the cells were washed with PBS, and the cells were digested with trypsin-EDTA. The cells in different experimental groups were collected into a EP tube, centrifuged at 1000 r/min for 5 min and the supernatant was discarded.
   6) To 100 µl of cells under each treatment factor, 200 µl of NaOH (1 mol/1) solution containing 10% dimethyl sulfoxide (DMSO) was added to lyse the cells.
   7) Completely dissolved the melanin particles in a water bath at 65°C for 20 min.
   8) The absorbance at 490 nm on the side of the microplate reader. Melanogenesis rate = (Experimental group B - Blank group B) / (Control group B - Blank group B) × 100%. The result was divided by the number of cells as an indicator for evaluating the level of melanin.

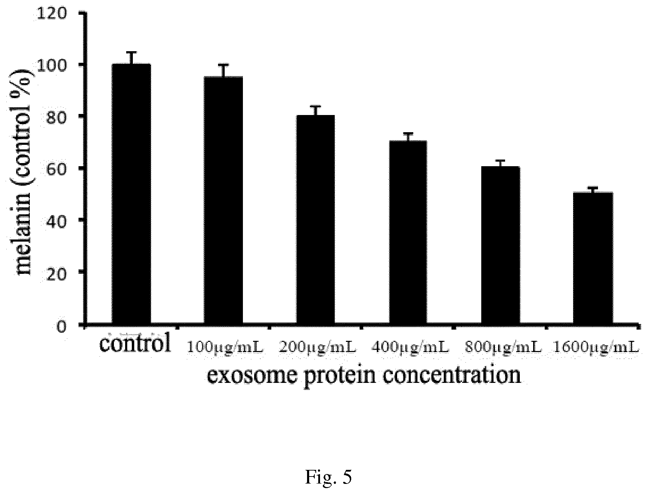

   Figure 5 shows the effect of exosomes on intracellular melanin content; the results showed that 800 µg/ml of exosomes from human perinatal mesenchymal stem cells can inhibit melanin synthesis, and the production rate of melanin was 60% of that of the control group.
(3) Effect of exosomes on tyrosinase activity in melanocytes
   1) 1) - 5) were the same as in step (2).
   6) 200 µl of 1% Triton X-100 solution was added, respectively.
   7) Disrupted cells at 4 °C by ultrasonic wave.
   8) 100 µl was pipetted and transferred to a 96-well plate, and 20 µl of 0.1% levodopa solution was added to each well.
   9) Shook for 5 min and incubated in a 37 °C incubator for 1 h.
   10) A490 value was measured on a spectrophotometer.

Tyrosine Activity rate = (Experimental group B - Blank group B) / (Control group B - Blank group B) × 100%. The result was divided by the number of cells as an indicator for evaluating the tyrosinase activity. The exosome at a concentration of 400ug/ml can inhibit tyrosine activity better.

In the present embodiment, the CCK-8 method was used to measure the effect of exosomes from human perinatal mesenchymal stem cells on the growth of human melanocytes, and the results show that the exosomes from human perinatal mesenchymal stem cells at ≤800 µg/ml had no obvious inhibition to human melanocytes, and no obvious symptoms of death were observed under the microscope. The cell viability of the experimental group did not show significant cell death compared with the control group, but the exosomes at a concentration of 800 µg/ml were able to inhibit the synthesis of melanin, inhibit the activity of intracellular tyrosinase, and has a good potential of whitening skin care.

The obtained exosomes can be used for local injection, or supplemented with hyaluronic acid and the like, and then injected with a microneedle, or applied externally to the skin.

The positive effects of the method provided by the present embodiment compared with the prior art method are as follows:
(1) A large number of exosomes secreted by human umbilical cord and placental stem cells.
(2) The contents of hepatocyte growth factor (HGF) and basic fibroblast growth factor (bFGF) contained in exosomes are increased, and the activity of exosomes is increased.
(3) The exosomes were extracted with polyethylene glycol in a final concentration of 10% and 8% consecutively, which improves the production of exosomes and simplifies the extraction process, compared with the conventional ultracentrifugation method.
(4) It can be applied widely, either can be applied in a form of hydrogel composition externally or locally by physiological saline.

The above description is only a preferred embodiment of the present disclosure, and is not intended to limit the present disclosure. Any modifications, equivalent substitutions, improvements, etc., which are included in the spirit and principle of the present disclosure, should be included within the scope of protection of the present disclosure.

## Claims

1. Use of a mesenchymal stem cell-derived exosome in a skin whitening preparations.

2. The use of a mesenchymal stem cell-derived exosome in a skin whitening preparation according to claim 1, **characterized in that**, the mesenchymal stem cells are human mesenchymal stem cells.

3. The use of a mesenchymal stem cell-derived exosome in a skin whitening preparation according to claim 2, **characterized in that**, the mesenchymal stem cells are originated from human perinatal umbilical cord or placenta.

4. The use of a mesenchymal stem cell-derived exosome in a skin whitening preparation according to claim 3, **characterized in that**, the concentration of exosomes is 800 µg/ml or more.

5. The application of the mesenchymal stem cell-derived exosome in a skin whitening preparation according to claim 4, **characterized in that**, the skin whitening preparation is an externally applied preparation or a preparation for local injection or microneedle injection.

6. The use of a mesenchymal stem cell-derived exosome in a skin whitening preparation according to claim 5, **characterized in that**, the mesenchymal stem cells are prepared by the following method:
the placenta of the full-term fetus delivered by caesarean section is taken, the chorion from the placenta is separated under sterile conditions, RBCs are rinsed off with PBS, the chorion is cut into tissue blocks with a size of 10-20 mm³, to which collagenase digestion solution is added, and is magnetically stirred and digested at a temperature around 37°C for a period of time; after the digested tissue suspension is filtered through a 100 mesh filter, the filtrate is added to a centrifuge tube containing a small amount of 1×PBS, and the remaining tissue is further magnetically stirred and digested by trypsin at a temperature around 37°C for a certain period of time, the digested suspension is filtered through a 100-mesh filter, and the filtrate is collected into a centrifuge tube, to which serum is added to terminate digestion; after the tissue block has almost been digested completely, it is centrifuged, the supernatant is discarded carefully, and the cells obtained by centrifugation are collected; some cell samples are taken and counted, then inoculated to DMEM-LG medium containing 10% FBS at a density of 2×10⁶/cm²; after cultivation in an incubator at a temperature around 37°C, saturated humidity, and 5% CO₂ for several days, culturing medium is replaced for the first time, to remove unattached cells; then medium is changed in a regular interval, and the morphology and growth of the cells are observed under the microscope daily; when a confluence of 80% to 90% is reached, the digested cells are subcultured;
the mesenchymal stem cell derived-exosomes are prepared by the following methods:
3-6 passages of human placenta mesenchymal stem cells are cultured in DMEM medium supplemented with 10% fetal bovine serum; when the cell confluence reaches 70-80%, cell cultivation continues till the cell confluence reaches 90%; supernatant is collected then centrifuged at 2000G for 20 min to remove cells and debris, then is filtered through a 0.22 µm sterile filter into a centrifuge tube, then polyethylene glycol 8000 solution with a final concentration of 10% is added into the centrifuge tube, vortexed and mixed homogenously, and incubated at 4°C overnight; then after centrifugation at 4000G for 20 min, supernatant is poured out, and sediment is resuspended in PBS solution with a volume of 1/100-1/50 of that of original supernatant, then is incubated in the polyethylene glycol of a final concentration of 8% for 1h again, and after centrifugation, sediment is resuspended in PBS, dispensed and stored at -80°C.
